# EUROPEAN PATENT SPECIFICATION

(11) **EP 3 682 822 B1**
(45) Date of publication and mention of the grant of the patent: **08.05.2024**
(21) Application number: 19152500.5
(22) Date of filing: 18.01.2019
(51) Int. Cl.: A61B 17/225

(54) **COMBINED SHOCKWAVE AND ULTRASOUND SOURCE**
KOMBINIERTE STOSSWELLEN- UND ULTRASCHALLQUELLE
SOURCE COMBINÉE D'ONDES DE CHOC ET D'ULTRASONS

(43) Date of publication of application: 22.07.2020
(73) Proprietor: Storz Medical AG, 8274 Tägerwilen (CH)
(72) Inventor: NOVAK, Pavel, 8234 Stetten (CH)
(74) Representative: Lohr, Jöstingmeier & Partner

(56) References cited:
- CN-A- 108 720 897
- KR-A- 20090 117 208
- US-A1- 2011 054 363

## Description

### Field of the invention

The invention relates to a device for generating of acoustic shockwaves together with ultrasound waves. The generated waves may be used to fragment stones in human or animal bodies.

### Description of the related art

A shockwave source is disclosed in US 5,058,569. A coil is mounted on a cylindrical support. When a high voltage pulse is applied to the coil, further causing a high current flow in a surrounding cylindrical membrane, the cylindrical membrane expands in radial direction causing a cylindrical pressure wave in the medium surrounding the coil. Such a medium preferably is a liquid like water. The generated cylindrically shaped pressure wave is deflected and focused by a paraboloidal reflector to a focus point in an axial direction of the coil. On its way to the focal area and due to superposition in the focal area the pressure wave steepens up and becomes a shock wave.

In EP 1 651 120 B1, a shockwave generating system comprising two shockwave generators is disclosed.

In general, all the shockwave devices can break large stones into smaller particles. Ultrasound devices are able to further reduce the particle size. Therefore, a combined treatment with shockwaves and ultrasound leads to significantly better results than the treatment with either shockwaves or ultrasound alone. In practice, it is difficult to apply a shockwave source first, remove the shockwave source and apply an ultrasound source in a next step. This is too complex and time-consuming. Furthermore, between the two steps the smaller particles of the stone generated by the shockwave drift away from the focus spot, such that focusing on the individual separated particles is very difficult.

EP 2 529 678 B1 discloses a shockwave apparatus comprising two coaxially arranged shockwave sources. A first shockwave source is driven by a coil, while a second shockwave source is a ballistic shockwave source.

EP1651120 discloses two coaxially arranged shockwave sources.

US 2011/0054363 A1 discloses a device that can deliver Lithotripsy therapy and Histotripsy therapy to a patient.

This combined solution allows the treatment with two different signals at the same time or immediately after each other, which leads to a significant reduction of particle size.

These embodiments have another disadvantage, in that the center of the coil is occupied for a second source and cannot be used for an ultrasound, or x-ray localization device. Therefore, an off-axis location device has to be used which makes the whole lithotripter more complex and difficult to use.

Further disadvantage is that the penetration depth of radial pressure wave is significantly smaller compared to the focusing electromagnetic shock wave source.

### Summary of the invention

The problem to be solved by the invention is to further reduce the size of the remaining stone particles. Another object of the invention is to reduce damage of body tissue and specifically to reduce hematoma. Another object of the invention is to provide a solution which allows the use of an on-axis ultrasound location device. Another object of the invention is to keep the source as simple as possible.

Solutions of the problem are described in the independent claims. The dependent claims relate to further improvements of the invention.

Long term tests have shown that a combined shockwave and ultrasound source provides significantly better results compared to dual shockwave sources. The particle size can further be reduced in shorter time.

A combined shockwave and ultrasound source includes a transducer connected to a power generator. The transducer may further comprise a coil and a reflector. The coil has preferably a cylindrical shape having a center axis. The coil may be mounted with its center axis at the center axis of a concave reflector, which preferably has a rotationally symmetric design. It may have a shape of rotational paraboloid. Alternate transducers of the present invention may include a flat coil mounted arranged under an acoustic lens.

A power generator connected to the transducer generates a high power signal. When such a high power signal is applied to the transducer, it generates a wave, guided, reflected or directed to a focus area. If the transducer comprises a cylindrical coil, the cylindrical coil generates a cylindrical wave propagating in a radial direction of the coil and preferably being symmetrical to the center axis. This wave is reflected by the reflector towards a focus area, which also may be a focus point. Preferably, the focus area is on the center axis and distant from the coil. The distance may be in a range between 2 cm and 25 cm depending on the design of the reflector and the coil to leave sufficient space for positioning a patient such that a stone to be disintegrated.

The power generator preferably provides a signal in the ultrasound range from 20 kHz to 1 MHz. This signal may be a continuous signal with a duration of 10 ms to 100 s, most preferably in a range of 100 ms to 5 s. The ultrasound signal has an amplitude adapted to the coil, such that a power of 1 W to 1 kW is coupled into the coil. The ultrasound signal may be provided at the same time with the shockwave signals or between the shockwave signals. It may even completely fill the gap between shockwave pulses.

The power generator may further generate a shockwave signal comprising one or a plurality of high voltage pulses having a voltage in a range from 100 V to 25 kV, preferably 1 kV to 10 kV, and having a duration in a range of 10 ns to 10 ms.

Preferably, the shockwave and the ultrasound signal are not applied simultaneously to the coil. There is either a shockwave or an ultrasound signal applied.

A control unit may be provided to control the combined shockwave and ultrasound generator and to provide the timing and preferably the signal amplitude of the power output to the coil generating shockwave or ultrasound waves.

In an embodiment, an ultrasound generator is provided for generating an ultrasound signal and a shockwave generator is provided for generating a shockwave signal. The ultrasound signal and the shockwave signal are coupled to a combiner which leads the signals to the coil. The combiner either comprises an amplifier to amplify the signals, or provides a circuit for isolating the ultrasound generator from the shockwave generator and vice versa, such that a shockwave signal from a shockwave generator does not disturb or destroy the ultrasound generator, and vice versa.

A control unit may be provided to control the ultrasound generator and the shockwave generator as well as the combiner.

In another embodiment, the coil is connected to an ultrasound generator. Furthermore, an auxiliary coil is provided and connected to a shockwave generator. Preferably, both coils are arranged coaxially, and most preferably they are held by the same coil carrier. Although this embodiment requires a somewhat more complex coil, the ultrasound generator and the shockwave generator may be electrically insulated from each other, which simplifies the circuit design significantly.

### Description of Drawings

In the following the invention will be described by way of example, without limitation of the general inventive concept, on examples of embodiment with reference to the drawings.
Figure 1 shows a sectional view of an embodiment.
Figure 2 shows a basic electrical schematic diagram.
Figure 3 shows an embodiment with separated ultrasound and shockwave generators.
Figure 4 shows a shockwave generating circuit in more detail.
Figure 5 shows a self-oscillating ultrasound generating circuit in more detail.
Figure 6 shows a combined circuit in more detail.
Figure 7 shows an embodiment with two coils.
Figure 8 shows an embodiment of a piezo transducer.
Figure 9 shows an embodiment of a flat coil transducer.

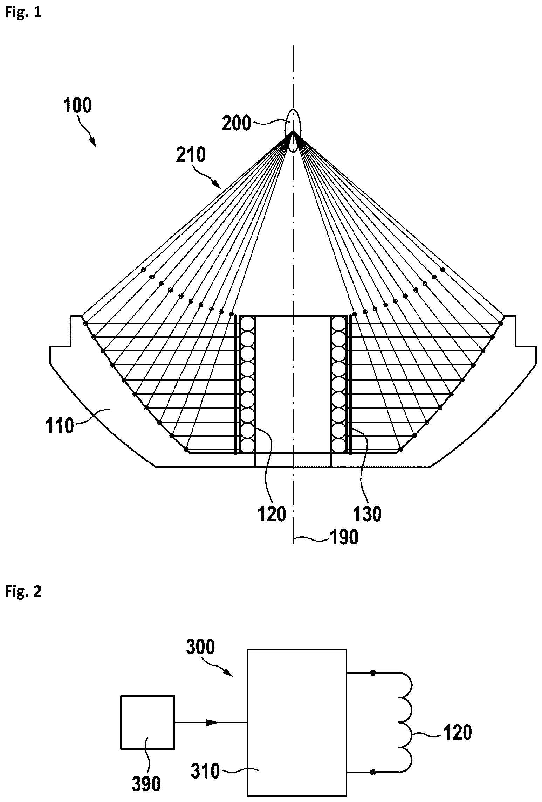

In Figure 1, a sectional view of an embodiment is shown. A shockwave and ultrasound source 100 comprises a coil 120 surrounded by a cylindrical membrane 130 held within a concave reflector 110. The coil with the membrane and the reflector preferably are rotationally symmetrical and have a common center axis 190. The coil preferably has a cylindrical shape which may be defined by a cylindrical body on which the windings of a conductive wire are held. Alternatively, the coil may be a flat coil with a flat membrane in front of it. The reflector preferably has a paraboloid shape such that a cylindrical wave generated by a cylindrical membrane is deflected to a focus area 200 which preferably is on the common center axis 190. The Figure symbolizes the wave propagation 210 from the coil to the focus area. The reflector may be optimized such that a specific shape of a focus area is obtained. A preferred focus area may be a comparatively small focus point, preferably having a diameter of 10 mm or less. In this embodiment, the coil has a hollow shape thus leaving space for an ultrasound location device or any other location device.

In Figure 2, a basic electrical schematic diagram is shown. The coil 120 is connected to a signal generator 300. This signal generator 300 preferably comprises a combined shockwave and ultrasound generator 310, which further may be controlled by a control unit 390. The combined shockwave and ultrasound generator generates signals with a suitable signal shape, frequency and power to operate the coil such that it generates shockwaves and ultrasound waves. The voltage for shockwaves may be in a range from 100 V to25 kV, preferably 1 kV to 10 kV, and having a duration in a range of 10 ns to 10 ms. The power for ultrasound may be in an ultrasound range from 20 kHz to 1 MHz. This signal may be a continuous signal with a duration of 10 ms to 100 s, most preferably in a range of 100 ms to 5 s. The ultrasound signal has an amplitude adapted to the coil, such that a power one 1 W to 1 kW is coupled into the coil. The ultrasound signal may be provided at the same time with the shockwave signals or between the shockwave signals. It may even completely fill the gap between shockwave pulses.

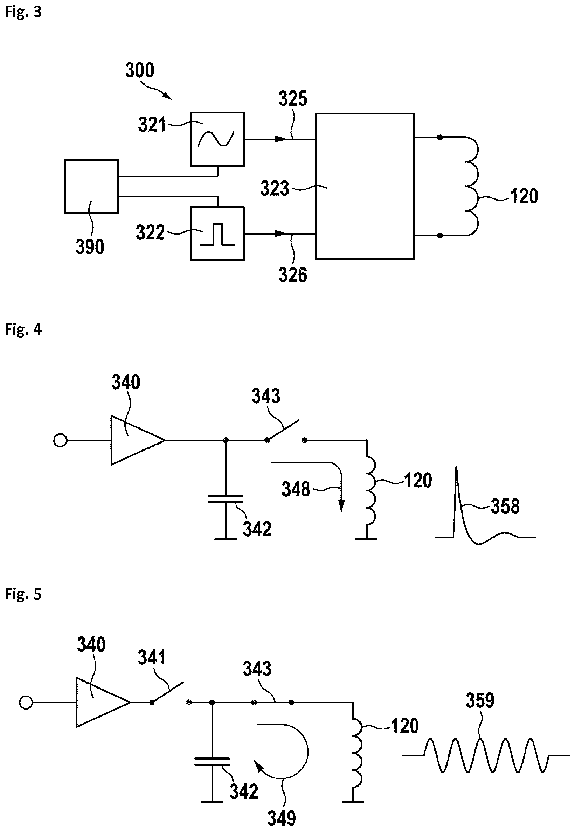

In Figure 3, an embodiment with separated ultrasound and shockwave generators is shown. An ultrasound generator 321 generates a wave form corresponding to the ultrasound waves later generated by the coil. A shockwave generator 322 generates a shockwave signal corresponding to the shockwaves which are later generated by the coil. The ultrasound generator 311 and the shockwave generator 322 may be controlled by a control unit 390. The ultrasound signal 325 of ultrasound generator 321 and the shockwave signal 326 of shockwave generator 322 are combined by a combiner 323 which further may be controlled by control unit 390. There may be two different embodiments of the combiner. In a first embodiment, the ultrasound generator 321 and the shockwave generator 322 only provide low power signals providing the required wave form. These low power signals are amplified in the combiner such that they have the required amplitude and power such that the coil may generate the required ultrasound waves and shockwaves. In another embodiment, the ultrasound generator 321 and the shockwave generator 322 provide ultrasound signals 325 and shockwave signals 326 with sufficient power to directly drive the coil. In this embodiment, the combiner simply combines the signals into a common signal for the coil. It is preferred if the combiner electrically insulates the ultrasound generator 321 from the shockwave generator 322 such that for example when the shockwave generator 322 generates a shockwave signal, this signal does not damage the ultrasound generator 321, and vice versa. In a most simple embodiment, the combiner may include a switch or a relay which switches only one of the sources to the coil.

Figure 4 shows a shockwave generating circuit in more detail. The circuit comprises a high voltage source 340, a capacitor 342 and a first switch 343. The capacitor is charged by the high voltage source until it holds a voltage and therefore energy sufficient for a shockwave pulse. After the charging level is reached, first switch 343 is closed for a short time such that the capacitor is discharged through coil 120 generating a shockwave pulse. The pulse current is indicated by arrow 348. The resulting coil current is symbolized by waveform 358. The closing time preferably is between 20% and 200% of the shockwave pulse time. In a further embodiment, a diode is in series with the first switch 343 in a direction, such that current can flow from the capacitor to the coil but not from the coil into the capacitor.

Figure 5 shows a self-oscillating ultrasound generating circuit in more detail. The circuit comprises a high voltage source 340 connected by a second witch 341 to a capacitor 342 and a first switch 343 in series with the coil 120. The capacitor is charged by the high voltage source until it holds a voltage and therefore energy sufficient for a ultrasound signal. After the charging level is reached, first switch 343 is closed for the duration of the ultrasound signal, such that the capacitor forms a resonance circuit together with the coil 120 generating an ultrasound signal. The resonance current is indicated by arrow 349. The resulting coil current is symbolized by waveform 359. Further, in order to maintain the amplitude of the ultrasound signal, the switch 341 may be repetitively closed to deliver energy needed to compensate for the energy dissipation in the oscillating circuit.

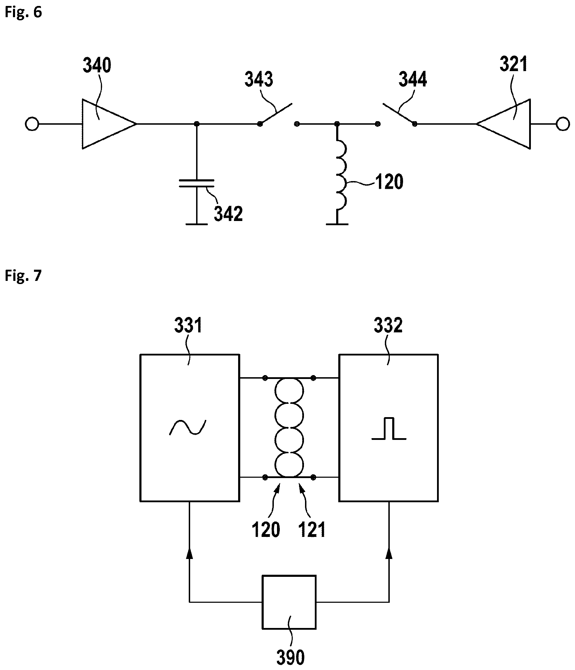

Figure 6 shows a combined circuit in more detail. The left side of the coil is the same as in figure 4, comprising a high voltage source 340, a capacitor 342 and a first switch 343. At the right side there is an ultrasound generator 321 connected by a third switch 344 to the coil. This third switch may be open, if shockwaves are generated and it may be closed, if an ultrasound signal has to be generated. An ultrasound signal may be directly coupled from ultrasound generator 321 into coil 120. In another embodiment, a resonance circuit with closed switch 343 formed by capacitor 342 and coil 120 may be used to generate the ultrasound signal. The ultrasound generator 321 may be used to provide sufficient energy to compensate for oscillating losses in the circuit. In this embodiment, it may be useful to have a first switch 341 in an open state as shown in the previous figure to isolate the high voltage source 340 from the resonance circuit.

In Figure 7, an embodiment with two coils which preferably are interleaved, is shown. Coil 120 may be connected to an ultrasound generator 331, and an auxiliary coil 121 may be provided which is connected to shockwave generator 332. Both generators may be controlled by a control circuit 390. It is preferred, if the coil 120 and the auxiliary coil 121 are arranged coaxially with their center axis.

Most preferably, the coils are on the same cylindrical base. Using separate coils results in a somewhat more complex mechanical design of the coil assembly, but provides an electrical insulation of the ultrasound generator 331 and the shockwave generator 332.

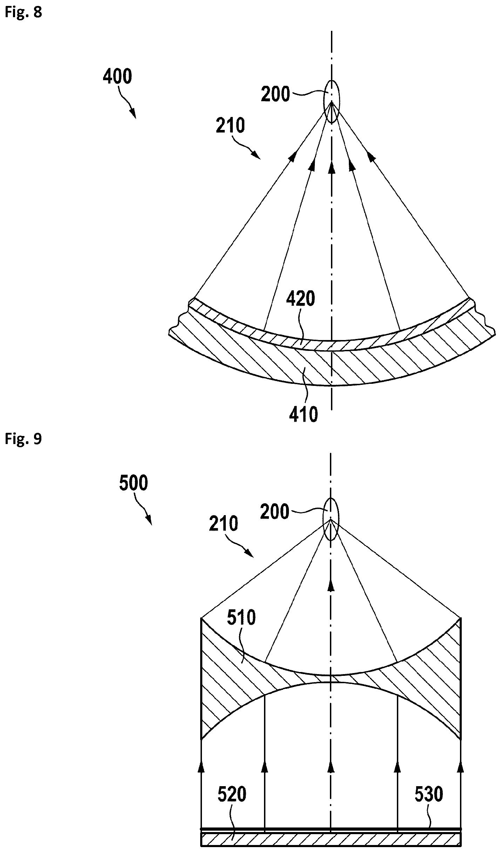

The embodiment of Figure 8 is not according to the invention and is present for illustration purposes only.

Figure 8 shows an embodiment of a piezo transducer 400. Here, a plurality of piezo elements 420 are held by a body 410 in a sphere-shaped arrangement, such that a plurality of waves generated by the plurality of piezo elements is focused into focus area 200.

Figure 9 shows a flat coil transducer 500, including a flat coil 520, a flat membrane 530 and an acoustic lens 510 to focus a planar wave generated by the flat coil with the flat membrane into focus area 200.

### List of reference numerals

- 100: shockwave and ultrasound source
- 110: reflector
- 120: coil
- 121: auxiliary coil
- 130: cylindrical membrane
- 190: center axis
- 200: focus area
- 210: wave propagation
- 300: power generator
- 310: combined shockwave and ultrasound generator
- 321: ultrasound generator
- 322: shockwave generator
- 323: combiner
- 325: ultrasound signal
- 326: shockwave signal
- 331: ultrasound generator
- 332: shockwave generator
- 340: high voltage source
- 341: first switch
- 342: capacitor
- 343: second switch
- 344: third switch
- 348: pulse current
- 349: oscillating current
- 358: shockwave pulse current waveform
- 359: ultrasound oscillating current waveform
- 390: control unit
- 400: piezo transducer
- 410: body
- 420: piezo elements
- 500: flat coil transducer
- 510: acoustic lens
- 520: flat coil
- 530: flat membrane

## Claims

1. Apparatus (100) for generating focused shockwaves and ultrasound waves comprising a transducer (110, 120) connected to a power generator (300), the power generator (300) comprises a combined shockwave and ultrasound generator (310) for providing ultrasound signals (325) and shockwave signals (326) to the transducer (110, 120) such that the transducer (110, 120) generates ultrasound waves and shockwaves,
**characterized in, that**
the transducer comprises a cylindrical coil (120) surrounded by a cylindrical membrane (130) held within a concave reflector (110).

2. Apparatus (100) for generating focused shockwaves and ultrasound waves comprising a transducer (110, 120) connected to a power generator (300), the power generator (300) comprises a combined shockwave and ultrasound generator (310) for providing ultrasound signals (325) and shockwave signals (326) to the transducer (110, 120) such that the transducer (110, 120) generates ultrasound waves and shockwaves,
**characterized in, that**
the transducer comprises a flat coil (520) with a flat membrane (530) arranged under an acoustic lens (510).

3. Apparatus (100) according to claim 1,
**characterized in, that**
the concave reflector (110) defines a center axis (190) and
the cylindrical membrane (130) is arranged with its center axis at the reflector center axis (190).

4. Apparatus (100) according to any of the previous claims,
**characterized in, that**
the power generator (300) is configured for alternatingly providing ultrasound signals (325) and shockwave signals (326).

5. Apparatus (100) according to any of the previous claims,
**characterized in, that**
the power generator (300) comprises an ultrasound generator (321) for providing an ultrasound signal (325) and a shockwave generator (322) for providing a shockwave signal (326), wherein the ultrasound generator (321) and the shockwave generator (322) are coupled via a combiner (323) to the coil.

6. Apparatus (100) according to claim 5,
**characterized in, that**
the combiner (323) is a switch.

7. Apparatus (100) according to claim 5,
**characterized in, that**
the combiner (323) comprises a power amplifier.

8. Apparatus (100) according to any of the previous claims,
**characterized in, that**
a control unit (390) is provided for controlling at least the signal generator (300).

9. Apparatus (100) according to claim 1,
**characterized in, that**
the coil (120) is connected to an ultrasound generator (331) and an auxiliary coil (121) is provided which is connected to a shockwave generator (332).

## Patentansprüche

1. Vorrichtung (100) zum Erzeugen fokussierter Stoßwellen und Ultraschallwellen, umfassend einen Wandler (110, 120), der mit einem Leistungsgenerator (300) verbunden ist, wobei der Leistungsgenerator (300) einen kombinierten Stoßwellen- und Ultraschallgenerator (310) zum Bereitstellen von Ultraschallsignalen (325) und Stoßwellensignalen (326) an den Wandler (110, 120) umfasst, so dass der Wandler (110, 120) Ultraschallwellen und Stoßwellen erzeugt,
**dadurch gekennzeichnet, dass**
der Wandler eine zylindrische Spule (120) umfasst, welche von einer zylindrischen Membran (130) umgeben ist, die innerhalb eines konkaven Reflektors (110) gehalten wird.

2. Vorrichtung (100) zum Erzeugen fokussierter Stoßwellen und Ultraschallwellen, umfassend einen Wandler (110, 120), der mit einem Leistungsgenerator (300) verbunden ist, wobei der Leistungsgenerator (300) einen kombinierten Stoßwellen- und Ultraschallgenerator (310) zum Bereitstellen von Ultraschallsignalen (325) und Stoßwellensignalen (326) an den Wandler (110, 120) umfasst, so dass der Wandler (110, 120) Ultraschallwellen und Stoßwellen erzeugt,
**dadurch gekennzeichnet, dass**
der Wandler eine flache Spule (520) mit einer flachen Membran (530) umfasst, welche unter einer akustischen Linse (510) angeordnet ist.

3. Vorrichtung (100) nach Anspruch 1,
**dadurch gekennzeichnet, dass**
der konkave Reflektor (110) eine Mittelachse (190) definiert und
die zylindrische Membran (130) mit ihrer Mittelachse auf der Reflektor-Mittelachse (190) angeordnet ist.

4. Vorrichtung (100) nach einem der vorhergehenden Ansprüche,
**dadurch gekennzeichnet, dass**
der Stromerzeuger (300) konfiguriert ist, um abwechselnd Ultraschallsignale (325) und Stoßwellensignale (326) bereitzustellen.

5. Vorrichtung (100) nach einem der vorhergehenden Ansprüche,
**dadurch gekennzeichnet, dass**
der Leistungsgenerator (300) einen Ultraschallgenerator (321) zum Bereitstellen eines Ultraschallsignals (325) und einen Stoßwellengenerator (322) zum Bereitstellen eines Stoßwellensignals (326) umfasst, wobei der Ultraschallgenerator (321) und der Stoßwellengenerator (322) über einen Kombinierer (323) an die Spule gekoppelt werden.

6. Vorrichtung (100) nach Anspruch 5,
**dadurch gekennzeichnet, dass**
der Kombinierer (323) ein Schalter ist.

7. Vorrichtung (100) nach Anspruch 5,
**dadurch gekennzeichnet, dass**
der Kombinierer (323) einen Leistungsverstärker umfasst.

8. Vorrichtung (100) nach einem der vorhergehenden Ansprüche,
**dadurch gekennzeichnet, dass**
eine Steuereinheit (390) zur Steuerung zumindest des Signalgenerators (300) vorgesehen ist.

9. Vorrichtung (100) nach Anspruch 1,
**dadurch gekennzeichnet, dass**
die Spule (120) mit einem Ultraschallgenerator (331) verbunden ist und eine Hilfsspule (121) vorgesehen ist, die mit einem Stoßwellengenerator (332) verbunden ist.

## Revendications

1. Appareil (100) pour générer des ondes de choc et des ondes ultrasonores focalisées, comprenant un transducteur (110, 120) connecté à un générateur de puissance (300), le générateur de puissance (300) comprend un générateur combiné d'ondes de choc et d'ultrasons (310) pour fournir des signaux ultrasonores (325) et des signaux d'ondes de choc (326) au transducteur (110, 120) de telle sorte que le transducteur (110, 120) génère des ondes ultrasonores et des ondes de choc,
**caractérisé en ce que**
le transducteur comprend une bobine cylindrique (120) entourée d'une membrane cylindrique (130) maintenue au sein d'un réflecteur concave (110).

2. Appareil (100) pour générer des ondes de choc et des ondes ultrasonores focalisées, comprenant un transducteur (110, 120) connecté à un générateur de puissance (300), le générateur de puissance (300) comprend un générateur combiné d'ondes de choc et d'ultrasons (310) pour fournir des signaux ultrasonores (325) et des signaux d'ondes de choc (326) au transducteur (110, 120) de telle sorte que le transducteur (110, 120) génère des ondes ultrasonores et des ondes de choc,
**caractérisé en ce que**
le transducteur comprend une bobine plate (520) avec une membrane plate (530) agencée sous une lentille acoustique (510).

3. Appareil (100) selon la revendication 1,
**caractérisé en ce que**
le réflecteur concave (110) définit un axe central (190) et
la membrane cylindrique (130) est agencée avec son axe central au niveau de l'axe central de réflecteur (190).

4. Appareil (100) selon l'une quelconque des revendications précédentes,
**caractérisé en ce que**
le générateur de puissance (300) est configuré pour fournir alternativement des signaux ultrasonores (325) et des signaux d'ondes de choc (326).

5. Appareil (100) selon l'une quelconque des revendications précédentes,
**caractérisé en ce que**
le générateur de puissance (300) comprend un générateur d'ultrasons (321) pour fournir un signal ultrasonore (325) et un générateur d'ondes de choc (322) pour fournir un signal d'ondes de choc (326), dans lequel le générateur d'ultrasons (321) et le générateur d'ondes de choc (322) sont couplés à la bobine par l'intermédiaire d'un combinateur (323).

6. Appareil (100) selon la revendication 5,
**caractérisé en ce que**
le combinateur (323) est un interrupteur.

7. Appareil (100) selon la revendication 5,
caractérisé en que
le combinateur (323) comprend un amplificateur de puissance.

8. Appareil (100) selon l'une quelconque des revendications précédentes,
**caractérisé en ce que**
une unité de commande (390) est prévue pour commander au moins le générateur de signaux (300).

9. Appareil (100) selon la revendication 1,
**caractérisé en ce que**
la bobine (120) est connectée à un générateur d'ultrasons (331) et une bobine auxiliaire (121) est prévue, laquelle est connectée à un générateur d'ondes de choc (332).
